# FASCICULE DE BREVET EUROPEEN

(11) **EP 1 458 413 B1**
(45) Date de publication et mention de la délivrance du brevet: **22.07.2009**
(21) Numéro de dépôt: 02801165.8
(22) Date de dépôt: 20.12.2002
(51) Int. Cl.: A61K 47/14

(54) **COMPOSITIONS PHARMACEUTIQUES A BASE DE DERIVES D AZETIDINE**
PHARMAZEUTISCHE ZUSAMMENSETZUNGEN AUF BASIS VON AZETIDINDERIVATEN
PHARMACEUTICAL COMPOSITIONS BASED ON AZETIDINE DERIVATIVES

(30) Priorité: 21.12.2001 FR 0116638
(43) Date de publication de la demande: 22.09.2004
(73) Titulaire: Aventis Pharma S.A., 92160 Antony (FR)
(72) Inventeur: COTÉ , Sophie, F-92160 ANTONY (FR); BOBINEAU, Valérie, F-91370 Verrieres le Buisson (FR); PERACCHIA, Maria-Teresa, F-75005 PARIS (FR)
(74) Mandataire: Morel-Pécheux, Muriel
(86) Numéro de dépôt international: PCT/FR2002/004514
(87) Numéro de publication internationale: WO 2003/053431

(56) Documents cités:
- WO-A-00/15609
- WO-A-01/64633
- WO-A-01/64634
- FR-A- 2 814 678

## Description

La présente invention concerne des formulations stables de dérivés d'azétidine.

Les dérivés d'azétidine utilisés dans les compositions pharmaceutiques selon l'invention peuvent être désignés par la formule générale (la) ou (Ib) ci-après : 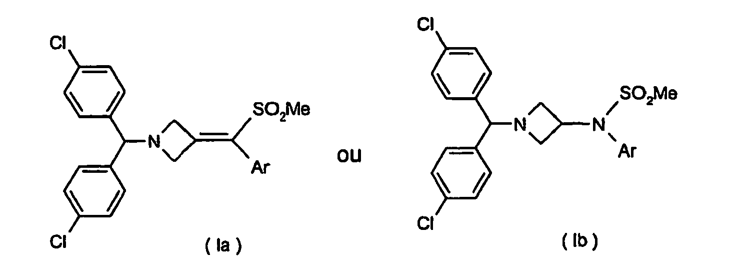 dans lesquelles Ar est un groupement aromatique ou hétéroaromatique éventuellement substitué par un ou plusieurs (C1-C4)alkyl, halogène, NO₂, CN, (C1-C4)alkoxy ou OH.
Dans la définition des dérivés d'azétidine ci-dessus, on entend notamment par groupement aromatique un groupement phényle, naphtyle, par groupement hétéroaromatique un groupement pyridyle, furyle, thiényle, thiazolyle, imidazolyle, oxazolyle et par halogène le fluor, le chlore, le brome ou l'iode.

Dans les demandes de brevets WO 00/15609, WO 01/64633, WO 01/64634, WO 99/01451 ont été décrits des dérivés d'azétidine de formule générale (la) ou (Ib) ainsi que leurs applications. Notamment, ces dérivés d'azétidine sont particulièrement intéressants pour leur forte affinité pour les récepteurs cannabinoïdes et particulièrement les récepteurs du type CB1.
Malheureusement les dérivés d'azétidine sont des produits très peu hydrosolubles.
Jusqu'à présent il était envisagé d'administrer les dérivés d'azétidine de formule générale (la) ou (Ib), notamment par voie orale, sous forme de comprimés dans des formulations comprenant entre autres de la cellulose, du lactose ainsi que d'autres excipients. Cependant de telles formulations ne sont pas toujours suffisamment bien adaptées à ces produits peu hydrosolubles du fait d'une trop faible biodisponibilité.

De nombreux documents décrivent des systèmes propres à solubiliser et/ou à améliorer la biodisponibilité de principes actifs hydrophobes. Cependant, les systèmes expérimentés se sont avérés jusqu'à présent inefficaces pour la préparation de compositions pharmaceutiques contenant des dérivés d'azétidine définis ci-dessus, stables, biodisponibles et dans lesquelles le dérivé d'azétidine est solubilisé à une concentration efficace.
Notamment, J. Pharm Sciences, 89(8), 967 (2000) et Pharmaceutical Technology Europe, p. 20, septembre 2000 mentionnent la formulation de principes actifs peu solubles dans l'eau, dans des triglycérides à chaines moyennes. Cependant les essais pratiqués avec des formulations à base de Miglyol®, ont donné des résultats insuffisants du point de vue de leur biodisponibilité.
Par ailleurs, la demande internationale WO 95/24893 décrit des compositions comprenant une huile digestible, un tensio-actif lipophile et un tensio-actif hydrophile destinées à la formulation de principes actifs hydrophobes et à l'amélioration de leur biodisponibilité. Malheureusement les dérivés d'azétidine ci-dessus se sont montrés trop peu biodisponibles dans ce type de formulation. Notamment, la formulation de tels dérivés d'azétidine dans un système Miglyol®/Capryol®/Cremophor® s'est également montrée insuffisante in vivo du point de vue pharmacocinétique.

Il a maintenant été trouvé, et c'est ce qui fait l'objet de la présente invention, que l'on peut préparer des compositions pharmaceutiques stables chimiquement et physiquement, comprenant un dérivé de formule générale (Ia) ou (Ib), éventuellement en association avec un autre principe actif capable de potentialiser les effets du dérivé d'azétidine de formule générale (Ia) ou (Ib), dans un système comprenant au plus 2 excipients principaux choisis parmi un agent tensio-actif non-ionique à caractère hydrophile capable de solubiliser le dérivé d'azétidine de formule générale (la) ou (Ib) et le cas échéant le principe actif potentialisant les effets du dérivé d'azétidine, et de provoquer la formation d'un système colloïdal, additionné éventuellement d'un second excipient de nature lipophile, stabilisant la formulation.

Selon l'invention, des compositions préférées comprennent :
■ au moins un principe actif de formule générale (Ia) ou (Ib),
■ éventuellement un autre principe actif capable de potentialiser les effets du dérivé d'azétidine de formule générale (Ia) ou (Ib),
■ un agent tensio-actif non-ionique à caractère hydrophile capable de solubiliser le dérivé d'azétidine de formule générale (Ia) ou (Ib) et le cas échéant le principe actif potentialisant les effets du dérivé d'azétidine, et capable de provoquer la formation d'un système colloïdal,
■ éventuellement un agent tensio-actif à caractère lipophile ayant une HLB inférieure à 10, et stabilisant la composition,
■ éventuellement des additifs complémentaires choisis parmi des agents stabilisants, des agents conservateurs, des agents permettant d'adapter la viscosité, ou des agents pouvant modifier par exemple les propriétés organoleptiques.

Selon l'invention, l'agent tensio-actif non-ionique à caractère hydrophile capable de solubiliser le dérivé d'azétidine de formule générale (Ia) ou (Ib) et le cas échéant le principe actif potentialisant les effets du dérivé d'azétidine, et capable de provoquer la formation d'un système colloïdal, peut être choisi parmi des agents solides ou semi-solides, fondant à basse température (T°C < 60°C), ou parmi des agents liquides, dont la HLB est comprise entre 10 et 20, tels que des glycérides de polyéthylène glycol et d'acides gras saturés.
Il est entendu que, dans la définition ci-dessus, les acides gras saturés peuvent contenir de 6 à 18 atomes de carbone, et que lesdits glycérides de polyéthylène glycol (PEG) et d'acides gras saturés peuvent être d'origine naturelle ou synthétique. A titre d'exemple, l'agent tensio-actif non-ionique à caractère hydrophile, peut être choisi parmi des agents comme le Labrasol® [caprylcaproyl macrogol-8 glycéride], les Gélucire® : Gélucire 44/14, Gélucire 50/13, [lauroyl (ou stéaroyl, palmitoyl) macrogol-32 glycéride].

Selon un mode préféré de l'invention, la composition peut comprendre également un agent un agent tensio-actif à caractère lipophile ayant une HLB inférieure à 10, et stabilisant la composition. Cet agent peut être choisi parmi les agents capables d'améliorer la solubilisation du dérivé d'azétidine de formule générale (Ia) ou (Ib) et si nécessaire, du principe actif associé. Selon l'invention, cet agent peut être choisi parmi les glycérides de polyéthylène glycol et d'acides gras, notamment d'acides gras insaturés, parmi les esters de polyéthylèneglycol et d'acides gras, ou parmi les esters d'acides gras et de sorbitol. Etant entendu que les acides gras ci-avant peuvent contenir de 6 à 18 atomes de carbone.
A titre d'exemple l'agent peut être choisi parmi l'acide oléique, parmi les Labrafil® [oléoyl (ou linoléoyl) macrogol-8 glycérides] par exemple le Labrafil M1944CS, le Capryol90® (monocaprylate de polyéthylène glycol), ou le Span 20® (monolaurate de sorbitol). La présente liste étant donnée à titre non limitatif.
Parmi les excipients cités ci-dessus plus particulièrement préférés sont notamment le Labrasol®, le Gélucire® ou le couple Labrafil®/Labrasol®.

Il a également été mis en évidence (mais non publié à la date de dépôt de la présente demande), que pour certains traitements comme par exemple l'obésité, il pouvait être avantageux d'administrer les dérivés d'azétidine de formule générale (Ia) ou (Ib) en même temps que la sibutramine qui provoque un effet de synergie dans la réduction de la consommation alimentaire.
La sibutramine et ses effets ont été décrits dans les références ci-après : WO 90/061110 ; D.H. RYAN et coll., Obesity Research, 3 (4), 553 (1995); H.C. JACKSON et coll., British Journal of Pharmacology, 121, 1758 (1997); G. FANGHANEL et Coll., Inter. J. Obes., 24 (2), 144 (2000); G.A. BRAY et coll., Obes. Res., 7(2), 189 (1999).
Par ailleurs pour d'autres traitements tels que la schizophrénie ou le traitement des désordres neurologiques comme la maladie de Parkinson, il peut être avantageux d'administrer les dérivés d'azétidine de formule générale (Ia) ou (Ib) en même temps qu'un ou plusieurs agents qui activent la neurotransmission dopaminergique dans le cerveau. Ces associations permettent de potentialiser les effets d'une monothérapie dopaminergique (lévodopa, agonistes dopaminergiques, et inhibiteurs d'enzymes), et permettent de réduire les effets secondaires, en particulier les dyskinésies.
Parmi les agonistes dopaminergiques, on peut notamment citer les produits suivants : bromocriptine (Novartis), cabergoline (Pharmacia Corp.) adrogolide (Abbott Laboratories), BAM-1110(Maruko Seiyaku Co Ltd), Duodopa^{®}(Neopharma), L-dopa, dopadose (Neopharma), CHF1512 (Chiesi), NeuroCell-PD (Diacrin Inc), PNU-95666 (Pharmacia & Upjohn), ropinirole (GlaxoSmithKline Beecham), pramipexole (Boehringer Ingelheim) rotigotine (Discovery Therapeutics, Lohmann Therapie System), spheramine (Titan Pharmaceuticals), TV1203 (Teva pharmaceutical), uridine (Polifarma).

Il est entendu que les compositions comprenant en outre un principe actif autre que le dérivé d'azétidine de formule générale (Ia) ou (Ib) et capable d'en potentialiser les effets, peuvent contenir un produit tel que défini dans les paragraphes ci-dessus et que lesdites compositions entrent dans le cadre de la présente invention.

Selon l'invention, le principe actif de formule générale (Ia) ou (Ib) représente de 0,01 à 70% en poids de la composition totale. De préférence il représente de 0,05 à 50% en poids et plus particulièrement encore, de 0,1 à 20% en poids de la composition totale.
Il est entendu que la posologie peut varier selon le degré ou la nature de l'affection à traiter. Ainsi, la quantité de produit actif dans une composition selon l'invention sera déterminée de telle manière qu'une posologie convenable puisse être prescrite. De ce fait la quantité de dérivé d'azétidine de formule générale (Ia) ou (Ib) varie en fonction de sa solubilité dans le mélange et également en fonction du dosage approprié pour le traitement des patients.
Chez l'homme, les doses journalières administrées par voie orale sont généralement comprises entre 0,1 et 100 mg de dérivé d'azétidine de formule générale (Ia) ou (Ib).
Il est entendu que, pour choisir le dosage le plus approprié, devront être pris en compte le poids du malade, son état de santé général, son âge et tous les facteurs qui peuvent influer sur l'efficacité du traitement. De préférence, les compositions sont préparées de telle façon qu'une dose unitaire contienne de 0,1 à 50 mg de produit actif.
Parmi les dérivés d'azétidine de formule générale (Ia) ou (Ib), plus particulièrement préférés sont les produits suivants :
■ 1-[bis(4-chlorophényl)méthyl]-3-[(3,5-difluorophényl)(méthylsulfonyl)méthylène] azétidine) ;
■ N-{1-[bis-(4-chlorophényl)méthyl]azétidin-3-yl}-N-pyrid-3-yl-méthylsulfonamide
■ N-{1-[bis-(4-chlorophényl)méthyl]azétidin-3-yl}-N-(3,5-difluorophényl)-méthylsulfonamide
■ N-{1-[bis-(4-chlorophényl)méthyl]-azétidin-3-yl}-N-(6-chloropyrid-2-yl)-méthylsulfonamide
■ N-{1-[bis-(4-chlorophényl)méthyl]azétidin-3-yl}-N-quinol-6-yl-méthylsulfonamide

Il est entendu que les compositions selon l'invention, contenant ces produits, sont particulièrement préférées.

Dans l'alternative où l'on introduit un second principe actif, les compositions peuvent comprendre 0,2 à 15 mg dans le cas où le produit associé est la sibutramine. Cependant cette quantité pourra éventuellement être plus faible et varier de 0,2 à 10 mg.
Dans le cas où le produit associé est la L-dopa, les compositions peuvent comprendre 100 à 300 mg de ce second principe actif, de préférence 250 mg.

L'agent tensio-actif non ionique, à caractère hydrophile capable de provoquer la formation d'un système colloïdal peut représenter de 20 à 100 % par rapport au poids total des excipients présents dans la composition, de préférence de 40 à 100 % et plus particulièrement de 60 à 100 %.

Le cas échéant, lorsque la composition contient aussi un agent de nature lipophile ayant une HLB inférieure à 10, la quantité de cet agent à faible HLB peut représenter de 0,1 à 60% par rapport au poids total des excipients présents dans la composition, et plus particulièrement de 1 à 40 %.

Lorsque la composition comprend en plus certains additifs complémentaires, ces derniers peuvent être des agents stabilisants, des agents conservateurs, des agents permettant d'adapter la viscosité, ou des agents pouvant par exemple modifier les propriétés organoleptiques.
Les agents stabilisants peuvent être par exemple des agents anti-oxydants notamment choisis parmi l'α-tocophérol, le palmitate d'ascorbyl, le BHT (butyl hydroxy toluène), le BHA (butyl hydroxy anisole), le propyl gallate ou l'acide malique par exemple ;
Les agents de conservation peuvent être à titre d'exemple choisis parmi le métabisulfite de sodium, le propylène glycol, l'éthanol ou la glycérine ;
Parmi les agents capables d'adapter la viscosité, peuvent être cités par exemple des lécithines, des phospholipides, l'alginate de propylène glycol, l'alginate de sodium ou la glycérine ;
Les agents capables de modifier les propriétés organoleptiques de la composition sont à titre d'exemple l'acide malique, l'acide fumarique, la glycérine, la vanilline ou le menthol.
Lorsque de tels additifs sont utilisés, ces derniers peuvent constituer de 0,001 % à 5 % en poids de la composition totale.

Selon l'invention, la composition pharmaceutique peut être obtenue par mélange le cas échéant des excipients principaux (après chauffage si nécessaire, dans le cas des excipients solides ou semi-solides), puis si nécessaire mélange avec les additifs complémentaires, puis addition du dérivé d'azétidine de formule générale (Ia) ou (Ib), et le cas échéant du principe actif capable de potentialiser les effets du dérivé d'azétidine de formule générale (Ia) ou (Ib), puis maintien sous agitation afin d'obtenir un mélange homogène.
La mise en oeuvre de ce procédé est décrite plus en détail ci-après dans les

### exemples.

Les compositions selon l'invention peuvent se présenter à l'état liquide, solide ou semi-pâteux.

Elles sont particulièrement adaptées pour une présentation sous forme de gélules ou de capsules molles, ou sous forme de solution buvable.

Les compositions selon l'invention sont particulièrement intéressantes du fait de leur bonne stabilité tant physique que chimique et de l'amélioration de la biodisponibilité qu'elles procurent lors de l'administration orale des dérivés d'azétidine de formule générale (Ia) ou (Ib).

Particulièrement préférées sont les compositions comprenant :
■ au moins un principe actif de formule générale (Ia) ou (Ib),
■ un agent tensio-actif non-ionique à caractère hydrophile capable de solubiliser le dérivé d'azétidine de formule générale (Ia) ou (Ib), et capable de provoquer la formation d'un système colloïdal,
■ éventuellement un agent tensio-actif à caractère lipophile ayant une HLB inférieure à 10, et stabilisant la composition,
■ éventuellement des additifs complémentaires choisis parmi des agents stabilisants, des agents conservateurs, des agents permettant d'adapter la viscosité, ou des agents pouvant modifier par exemple les propriétés organoleptiques.

Selon une autre alternative de l'invention, les compositions préférées telles que définies ci-dessus, contenant au moins un principe actif de formule générale (Ia) ou (Ib), peuvent être administrées avant, simultanément ou après l'administration d'un principe actif capable de potentialiser les effets du dérivé d'azétidine de formule générale (Ia) ou (Ib).
Il est entendu que les kits de présentation comprenant d'une part une composition préférée selon l'invention telle que définie ci-dessus et d'autre part une composition comprenant le principe actif capable de potentialiser les effets du dérivé d'azétidine de formule générale (Ia) ou (Ib), entrent aussi dans le cadre de la présente invention. Il est également entendu que les kits de présentation peuvent contenir à titre de composition capable de potentialiser les effets du dérivé d'azétidine de formule générale (Ia) ou (Ib), des compositions comprenant de la sibutramine, ou comprenant un agent activant la neurotransmission dopaminergique dans le cerveau.

Les exemples suivants, donnés à titre non limitatif, illustrent des compositions selon la présente invention.

### Exemple 1

Le mélange Labrasol/Labrafil M1944CS, ratio 60/40 (m/m) est préparé à température ambiante (20°C), par agitation magnétique pendant 15 minutes de 14,4 g de Labrasol et 9,6 g de Labrafil M1944CS dans un bécher. Une très bonne miscibilité est observée. 200 mg de 1-[bis(4-chlorophényl)méthyl]-3-[(3,5-difluorophényl)(méthylsulfonyl) méthylène] azétidine sont introduits dans un autre bécher, et complétés à 20 g par le mélange Labrasol / Labrafil M1944CS 60/40, pour obtenir une concentration finale de 10 mg/g de 1-[bis(4-chlorophényl)méthyl]-3-[(3,5-difluorophényl)(méthylsulfonyl) méthylène] azétidine. Le mélange des 3 constituants est maintenu sous agitation magnétique (300 rpm) à température ambiante pendant 2 heures afin d'obtenir une dissolution complète de la 1-[bis(4-chlorophényl)méthyl]-3-[(3,5-difluorophényl)(méthylsulfonyl) méthylène] azétidine. La solution obtenue est répartie par fraction de 1g dans des flacons en verre scellés et conservés à 5°C.

Une stabilité chimique et physique satisfaisante a été démontrée pendant 1 mois à 5°C.
Une amélioration des paramètres pharmacocinétiques d'au moins un facteur de 2,5 a été observée dans une comparaison à une composition de 1-[bis(4-chlorophényl)méthyl]-3-[(3,5-difluorophényl) (méthylsulfonyl) méthylène] azétidine dans du Miglyol 812®.

### Exemple 2

En opérant comme précédemment à l'exemple 1, mais à partir de 16,8 g de Labrasol et 7,2 g de Labrafil M1944CS pour fabriquer le mélange Labrasol/Labrafil M1944CS au ratio 70/30 (m/m), on prépare une composition contenant 200 mg de 1-[bis(4-chlorophényl) méthyl]-3-[(3,5-difluorophényl) (méthylsulfonyl) méthylène] azétidine complétés à 20 g par le mélange Labrasol / Labrafil M1944CS 70/30, pour obtenir une concentration finale de 10 mg/g de 1-[bis(4-chlorophényl) méthyl]-3-[(3,5-difluorophényl) (méthylsulfonyl) méthylène] azétidine.

Une stabilité chimique et physique satisfaisante a été démontrée pendant 1 mois à 5°C.

Cette composition a été testée dans un modèle in vitro, par comparaison à la composition de l'exemple 1. 400 mg de la composition ont été incubés dans 20 ml de milieu simulant le liquide gastrique (référence USP). Après une incubation de 2 heures à 37°C, un dosage CLHP a été effectué après filtration sur filtre 2µm, pour déterminer la stabilité colloïdale des formulations.
Le comportement de cette composition est équivalent au comportement de la composition de l'exemple 1.

### Exemple 3

En opérant comme précédemment à l'exemple 1, mais à partir de 19,2 g de Labrasol et 4,8 g de Labrafil M1944CS pour fabriquer le mélange Labrasol/Labrafil M1944CS au ratio 80/20 (m/m), on prépare une composition contenant 200 mg de 1-[bis(4-chlorophényl) méthyl]-3-[(3,5-difluorophényl) (méthylsulfonyl) méthylène] azétidine complétés à 20 g par le mélange Labrasol / Labrafil M1944CS 80/20, pour obtenir une concentration finale de 10 mg/g de 1-[bis(4-chlorophényl)méthyl]-3-[(3,5-difluorophényl)(méthylsulfonyl) méthylène] azétidine.

Une stabilité chimique et physique satisfaisante a été démontrée pendant 1 mois à 5°C.

Cette composition a été testée dans un modèle in vitro, par comparaison à la composition de l'exemple 1. 400 mg de la composition ont été incubés dans 20 ml de milieu simulant le liquide gastrique (référence USP). Après une incubation de 2 heures à 37°C, un dosage CLHP a été effectué après filtration sur filtre 2µm, pour déterminer la stabilité colloïdale des formulations.
Le comportement de cette composition est équivalent au comportement de la composition de l'exemple 1.

### Exemple 4

En opérant comme précédemment à l'exemple 1, mais à partir de 21,6 g de Labrasol et 2,4 g de Labrafil M1944CS pour fabriquer le mélange Labrasol/Labrafil M1944CS au ratio 90/10 (m/m), on prépare une composition contenant 200 mg de 1-[bis(4-chlorophényl) méthyl]-3-[(3,5-difluorophényl) (méthylsulfonyl) méthylène] azétidine complétés à 20 g par le mélange Labrasol / Labrafil M1944CS 90/10, pour obtenir une concentration finale de 10 mg/g de 1-[bis(4-chlorophényl) méthyl]-3-[(3,5-difluorophényl) (méthylsulfonyl) méthylène] azétidine.

Une stabilité chimique et physique satisfaisante a été démontrée pendant 1 mois à 5°C.

Cette composition a été testée dans un modèle in vitro, par comparaison à la composition de l'exemple 1. 400 mg de la composition ont été incubés dans 20 ml de milieu simulant le liquide gastrique (référence USP). Après une incubation de 2 heures à 37°C, un dosage CLHP a été effectué après filtration sur filtre 2µm, pour déterminer la stabilité colloïdale des formulations.
Le comportement de cette composition est équivalent au comportement de la composition de l'exemple 1.

### Exemple 5

En opérant comme précédemment à l'exemple 1, mais à partir de 24 g de Labrasol uniquement, on prépare une composition contenant 200 mg de 1-[bis(4-chlorophényl)méthyl]-3-[(3,5-difluorophényl)(méthylsulfonyl) méthylène] azétidine complétés à 20 g par du Labrasol, pour obtenir une concentration finale de 10 mg/g de 1-[bis(4-chlorophényl) méthyl]-3-[(3,5-difluorophényl) (méthylsulfonyl) méthylène] azétidine.

Une stabilité chimique et physique satisfaisante a été démontrée pendant 1 mois à 5°C.

Cette composition a été testée dans un modèle in vitro, par comparaison à la composition de l'exemple 1. 400 mg de la composition ont été incubés dans 20 ml de milieu simulant le liquide gastrique (référence USP). Après une incubation de 2 heures à 37°C, un dosage CLHP a été effectué après filtration sur filtre 2 µm, pour déterminer la stabilité colloïdale des formulations.
Le comportement de cette composition est équivalent au comportement de la composition de l'exemple 1.

### Exemple 6

En opérant comme précédemment à l'exemple 1, mais à partir de 24 g de Gélucire 44/14 en remplacement du mélange Labrasol / Labrafil M1944CS. Le Gélucire 44/14 est préalablement fondu vers 55°C. 200 mg de 1-[bis(4-chlorophényl)méthyl]-3-[(3,5-difluorophényl)(méthylsulfonyl)méthylène] azétidine sont introduits dans un bécher, et complètés à 20 g avec le Gélucire 44/14, pour obtenir une concentration finale de 10 mg/g de 1-[bis(4-chlorophényl) méthyl]-3-[(3,5-difluorophényl) (méthylsulfonyl) méthylène] azétidine. Le mélange des 2 constituants est maintenu sous agitation magnétique (300 rpm) à 50-55°C pendant 1 heure afin d'obtenir une dissolution complète de 1-[bis(4-chlorophényl) méthyl]-3-[(3,5-difluorophényl) (méthylsulfonyl) méthylène] azétidine. La masse est répartie dans des gélules qui sont stockées une nuit au congélateur à -20°C. L'enveloppe des gélules est ensuite séparée de la masse solide contenue à l'intérieur à l'aide d'un cutter. Les échantillons sont conservés dans des flacons en verre scellés à 5°C.

Une stabilité chimique satisfaisante a été démontrée pendant 1 mois à 5°C.

Cette composition a été testée dans un modèle in vitro, par comparaison à la composition de l'exemple 1. 400 mg de la composition ont été incubés dans 20 ml de milieu simulant le liquide gastrique (référence USP). Après une incubation de 2 heures à 37°C, un dosage CLHP a été effectué après filtration sur filtre 2µm, pour déterminer la stabilité colloïdale des formulations.
Le comportement de cette composition est équivalent au comportement de la composition de l'exemple 1.

### Exemple 7

Un mélange Labrasol / Labrafil M1944CS, ratio 60/40 (m/m) est préparé à température ambiante (20°C), par agitation magnétique pendant 15 minutes de 30 g de Labrasol et 20 g de Labrafil M1944CS dans un bêcher. Une très bonne miscibilité est observée. 20 mg de 1-[bis(4-chlorophényl)méthyl]-3-[(3,5-difluorophényl) (méthylsulfonyl) méthylène] azétidine sont introduits dans une fiole jaugée de 10 ml. Après avoir complété à 10 ml avec la quantité nécessaire de mélange Labrasol / Labrafil M1944CS 60/40, le mélange des 3 constituants est maintenu sous agitation magnétique (500 rpm) à température ambiante pendant 2 heures afin d'obtenir une dissolution complète de la 1-[bis(4-chlorophényl)méthyl]-3-[(3,5-difluorophényl)(méthylsulfonyl) méthylène] azétidine. La solution obtenue est répartie par fraction de 2,5 ml dans des flacons en verre scellés et conservés à 5°C.

Cette formulation à la concentration de 2 mg/ml de 1-[bis(4-chlorophényl)méthyl]-3-[(3,5-difluorophényl)(méthylsulfonyl) méthylène] azétidine a été utilisée pour réaliser des études de pharmacocinétique chez le singe après administration par voie orale à une dose de 1 mg/kg. Pour ce faire cette solution a été diluée au dixième dans du jus de pomme pour faciliter l'administration à l'animal. L'émulsion obtenue après dilution est stable physiquement et chimiquement pendant au moins une heure.

### Exemple 8

Un mélange Labrasol / Labrafil M1944CS, ratio 60/40 (m/m) est préparé à température ambiante (20°C), par agitation magnétique pendant 15 minutes de 30 g de Labrasol et 20 g de Labrafil M1944CS dans un bécher. Une très bonne miscibilité est observée. 20 mg de N-{1-[bis-(4-chlorophényl)méthyl]azétidin-3-yl}-N-(3,5-difluorophényl)-méthyl-sulfonamide sont introduits dans une fiole jaugée de 10 ml. Après avoir complété à 10 ml avec la quantité nécessaire de mélange Labrasol / Labrafil M1944CS 60/40, le mélange des 3 constituants est maintenu sous agitation magnétique (500 rpm) à température ambiante pendant 2 heures afin d'obtenir une dissolution complète de la N-{1-[bis-(4-chlorophényl)méthyl]azétidin-3-yl}-N-(3,5-difluorophényl)-méthyl-sulfonamide. La solution obtenue est répartie par fraction de 2,5 ml dans des flacons en verre scellés et conservés à 5°C.

Cette formulation à la concentration de 2 mg/ml de N-{1-[bis-(4-chlorophényl)méthyl]azétidin-3-yl}-N-(3,5-difluorophényl)-méthyl-sulfonamide a été utilisée pour réaliser des études de pharmacocinétique chez le singe après administration par voie orale à une dose de 1 mg/kg. Pour ce faire cette solution a été diluée au dixième dans du jus de pomme pour faciliter l'administration à l'animal. L'émulsion obtenue après dilution est stable physiquement et chimiquement pendant au moins une heure.

### Exemple 9

Un mélange Labrasol/Labrafil M1944CS, ratio 60/40 (m/m) est préparé à température ambiante (20°C), par agitation magnétique pendant 15 minutes de 30 g de Labrasol et 20 g de Labrafil M1944CS dans un bécher. Une très bonne miscibilité est observée. 10 mg de N-{1-[bis-(4-chlorophényl)méthyl]azétidin-3-yl}-N-pyrid-3-yl-méthyl-sulfonamide sont introduits dans une fiole jaugée de 10 ml. Après avoir complété à 10 ml avec la quantité nécessaire de mélange Labrasol / Labrafil M1944CS 60/40, le mélange des 3 constituants est maintenu sous agitation magnétique (500 rpm) à température ambiante pendant 2 heures afin d'obtenir une dissolution complète de la N-{1-[bis-(4-chlorophényl)méthyl]azétidin-3-yl}-N-pyrid-3-yl-méthyl-sulfonamide. La solution obtenue est répartie par fraction de 2,5 ml dans des flacons en verre scellés et conservés à 5°C.

Cette formulation à la concentration de 1mg/ml de N-{1-[bis-(4-chlorophényl)méthyl]azétidin-3-yl}-N-pyrid-3-yl-méthyl-sulfonamide a été utilisée pour réaliser des études de pharmacologie chez le rat après administration par voie orale à une dose de 1 mg/kg.

### Exemple 10

En opérant comme précédemment à l'exemple 9, mais à partir de 30 mg de N-{1-[bis-(4-chlorophényl)méthyl]azétidin-3-yl}-N-pyrid-3-yl-méthyl-sulfonamide complètés à 10 ml avec le mélange Labrasol / Labrafil M1944CS 60/40, on prépare une solution à 3 mg/ml de N-{1-[bis-(4-chlorophényl)méthyl]azétidin-3-yl}-N-pyrid-3-yl-méthylsulfonamide.

Cette formulation à la concentration de 3mg/ml de N-{1-[bis-(4-chlorophényl)méthyl] azétidin-3-yl}-N-pyrid-3-yl-méthyl-sulfonamide a été utilisée pour réaliser des études de pharmacologie chez le rat après administration par voie orale à une dose de 3 mg/kg.

### Exemple 11

En opérant comme précédemment à l'exemple 9, mais à partir de 50 mg de N-{1-[bis-(4-chlorophényl)méthyl]azétidin-3-y1}-N-pyrid-3-yl-méthyl-sulfonamide complètés à 5 ml avec le mélange Labrasol / Labrafil M1944CS 60/40, on prépare une solution à 10 mg/ml de N-{1-[bis-(4-chlorophényl)méthyl]azétidin-3-yl}-N-pyrid-3-yl-méthylsulfonamide.

Cette formulation à la concentration de 10 mg/ml de N-{1-[bis-(4-chlorophényl) méthyl] azétidin-3-yl}-N-pyrid-3-yl-méthyl-sulfonamide a été utilisée pour réaliser des études de pharmacologie chez le rat après administration par voie orale à une dose de 10 mg/kg.

## Revendications

1. - Composition pharmaceutique stable comprenant au moins un dérivé d'azétidine de formule générale : 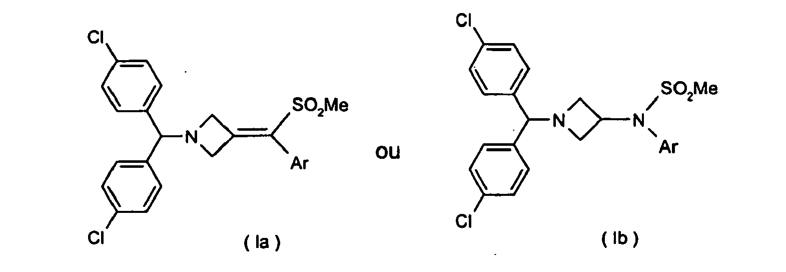 dans laquelle Ar est un groupement aromatique ou hétéroaromatique éventuellement substitué par un ou plusieurs (C1-C4)alkyle, halogène, NO₂, CN, (C1-C4)alkoxy ou OH, éventuellement en association avec un autre principe actif capable de potentialiser les effets du dérivé d'azétidine de formule générale (Ia) ou (Ib), dans un système comprenant au plus 2 excipients principaux choisis parmi un agent tensio-actif non-ionique à caractère hydrophile capable de solubiliser le dérivé d'azétidine de formule générale (Ia) ou (Ib) et le cas échéant le principe actif potentialisant les effets du dérivé d'azétidine, et capable de provoquer la formation d'un système colloïdal, additionné éventuellement d'un second excipient de nature lipophile, stabilisant la formulation.

2. - Composition pharmaceutique selon la revendication 1, **caractérisée en ce qu'**elle comprend :
■ au moins un dérivé d'azétidine de formule générale (Ia) ou (Ib), selon la revendication 1,
■ éventuellement un autre principe actif capable de potentialiser les effets du dérivé d'azétidine de formule générale (Ia) ou (Ib),
■ un agent tensio-actif non-ionique à caractère hydrophile capable de solubiliser le dérivé d'azétidine de formule générale (Ia) ou (Ib) et le cas échéant le principe actif potentialisant les effets du dérivé d'azétidine, et capable de provoquer la formation d'un système colloïdal,
■ éventuellement un agent tensio-actif à caractère lipophile ayant une HLB inférieure à 10, et stabilisant la composition,
■ éventuellement des additifs complémentaires choisis parmi des agents stabilisants, des agents conservateurs, des agents permettant d'adapter la viscosité, ou des agents pouvant modifier par exemple les propriétés organoleptiques.

3. - Composition pharmaceutique selon la revendication 1 ou 2, **caractérisée en ce qu'**elle comprend :
■ au moins un dérivé d'azétidine de formule générale (Ia) ou (Ib), selon la revendication 1,
■ un agent tensio-actif non-ionique à caractère hydrophile capable de solubiliser le dérivé d'azétidine le dérivé d'azétidine selon la revendication 1, et capable de provoquer la formation d'un système colloïdal,
■ éventuellement un agent tensio-actif à caractère lipophile ayant une HLB inférieure à 10, et stabilisant la composition,
■ éventuellement des additifs complémentaires choisis parmi des agents stabilisants, des agents conservateurs, des agents permettant d'adapter la viscosité, ou des agents pouvant modifier par exemple les propriétés organoleptiques.

4. - Composition pharmaceutique selon l'une des revendications 1 à 3, **caractérisée en ce que** le groupement aromatique du dérivé d'azétidine de formule générale (la) ou (Ib) est choisi parmi un groupement phényle ou naphtyle.

5. - Composition pharmaceutique selon l'une des revendications 1 à 4, **caractérisée en ce que** le groupement aromatique du dérivé d'azétidine de formule générale (Ib) est un groupement 3,5-difluorophényle.

6. - Composition pharmaceutique selon l'une des revendications 1 à 3, **caractérisée en ce que** le groupement hétéroaromatique est choisi parmi un groupement pyridyle, furyle, thiényle, thiazolyle, imidazolyle ou oxazolyle.

7. - Composition pharmaceutique selon l'une des revendications 1 à 6, **caractérisée en ce que** l'agent tensio-actif non-ionique à caractère hydrophile capable de solubiliser le dérivé d'azétidine de formule générale (Ia) ou (Ib) et le cas échéant le principe actif potentialisant les effets du dérivé d'azétidine, et capable de provoquer la formation d'un système colloïdal, est choisi parmi des glycérides de polyéthylène glycol et d'acides gras saturés, dont la HLB est comprise entre 10 et 20.

8. - Composition pharmaceutique selon la revendication 7, **caractérisée en ce que** les glycérides de polyéthylène glycol et d'acides gras saturés sont des glycérides de polyéthylène glycol et d'acides gras saturés contenant de 6 à 18 atomes de carbone.

9. - Composition pharmaceutique selon la revendication 7 ou 8, **caractérisée en ce que** les glycérydes peuvent être d'origine naturelle ou synthétique.

10. - Composition pharmaceutique selon l'une des revendications 1 à 9, **caractérisée en ce que** l'excipient à caractère lipophile, stabilisant la composition, est choisi parmi les glycérides de polyéthylène glycol et d'acides gras insaturés, parmi les esters de polyéthylèneglycol et d'acides gras ou parmi les esters d'acides gras et de sorbitol, ayant une HLB inférieure à 10.

11. - Composition pharmaceutique selon l'une des revendications 1 à 10, **caractérisée en ce que** le système comprenant au plus 2 excipients est constitué du Labrasol®, du Gélucire® ou du couple Labrafil®/Labrasol®.

12. - Composition pharmaceutique selon l'une des revendications 1 à 11, **caractérisée en ce que** le un principe actif dérivé d'azétidine, défini dans la revendication 1, est présent à raison de 0,01 à 70 % en poids de la composition totale.

13. - Composition pharmaceutique selon l'une des revendications 1 à 12, **caractérisée en ce que** l'agent tensio-actif non-ionique à caractère hydrophile capable de solubiliser le dérivé d'azétidine selon la revendication 1, et capable de provoquer la formation d'un système colloïdal, est présent à raison de 20 à 100 % par rapport au poids total des excipients dans la composition.

14. - Composition pharmaceutique selon l'une des revendications 1 à 13, **caractérisée en ce que** le cas échéant, agent à caractère lipophile, stabilisant la formulation, est présent à raison de 0,1 à 60 % par rapport au poids total des excipients dans la composition.

15. - Composition pharmaceutique selon l'une des revendications 1 à 14 **caractérisé en ce que :**
■ le principe actif dérivé d'azétidine est tel que défini à la revendication 5,
■ l'agent tensio-actif non-ionique à caractère hydrophile est le Labrasol,
■ l'agent tensio-actif à caractère lipophile ayant une HLB inférieure à 10 est le Labrafil M1944CS, et
■ le mélange Labrasol/Labrafil M1944CS est présent dans un ratio 60/40 (m/m) par rapport au poids total des excipients présents dans la composition.

16. - Composition pharmaceutique selon l'une des revendications 1 à 15 **caractérisé en ce qu'**elle se présente sous forme d'une capsule molle.

17. - Procédé de préparation d'une composition selon l'une des revendications 1 à 15, **caractérisée en ce que** l'on prépare le cas échéant le mélange de excipients principaux, après chauffage, si nécessaire, dans le cas des excipients solides ou semi-solides, puis le cas échéant, le mélange avec les additifs complémentaires, puis ajoute le dérivé d'azétidine défini selon la revendication 1, et le cas échéant le principe actif capable de potentialiser les effets du dérivé d'azétidine de formule générale (la) ou (Ib) défini dans la revendication 1, puis maintient sous agitation afin d'obtenir un mélange homogène.

18. - Kit de présentation contenant une composition selon la revendication 3 et une composition comprenant un principe actif capable de potentialiser les effets du dérivé d'azétidine défini dans la revendication 1.

19. - Kit de présentation selon la revendication 18 contenant une composition selon la revendication 3 et une composition comprenant de la sibutramine.

20. - Kit de présentation selon la revendication 18 contenant une composition selon la revendication 3 et une composition comprenant un agent activant la neurotransmission dopaminergique dans le cerveau.

## Claims

1. Stable pharmaceutical composition comprising at least one azetidine derivative of general formula: 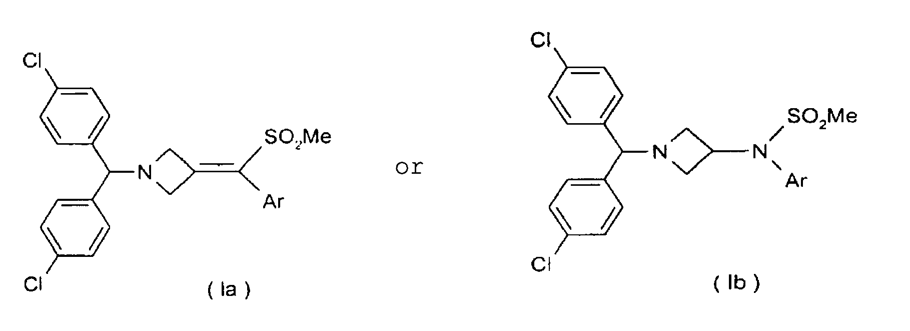 in which Ar is an aromatic or heteroaromatic group optionally substituted with one or more (C1-C4)alkyl, halogen, NO₂, CN, (C1-C4)alkoxy or OH groups, optionally in combination with another active ingredient capable of potentiating the effects of the azetidine derivative of general formula (Ia) or (Ib), in a system comprising at most 2 principal excipients chosen from a nonionic surfactant with a hydrophilic character capable of solubilizing the azetidine derivative of general formula (Ia) or (Ib) and, where appropriate, the active ingredient potentiating the effects of the azetidine derivative, and capable of causing the formation of a colloidal system, optionally supplemented with a second excipient of a lipophilic nature, stabilizing the formulation.

2. Pharmaceutical composition according to Claim 1, **characterized in that** it comprises:
• at least one azetidine derivative of general formula (Ia) or (Ib), according to Claim 1,
• optionally another active ingredient capable of potentiating the effects of the azetidine derivative of general formula (Ia) or (Ib),
• a nonionic surfactant with a hydrophilic character capable of solubilizing the azetidine derivative of general formula (Ia) or (Ib) and, where appropriate, the active ingredient potentiating the effects of the azetidine derivative, and capable of causing the formation of a colloidal system,
• optionally a surfactant with a lipophilic character having an HLB of less than 10, and stabilizing the composition,
• optionally additional additives chosen from stabilizing agents, preservatives, agents which make it possible to adjust the viscosity, or agents which can modify, for example, the organoleptic properties.

3. Pharmaceutical composition according to Claim 1 or 2, **characterized in that** it comprises:
• at least one azetidine derivative of general formula (Ia) or (Ib), according to Claim 1,
• a nonionic surfactant with a hydrophilic character capable of solubilizing the azetidine derivative according to Claim 1, and capable of causing the formation of a colloidal system,
• optionally a surfactant with a lipophilic character having an HLB of less than 10, and stabilizing the composition,
• optionally additional additives chosen from stabilizing agents, preservatives, agents which make it possible to adjust the viscosity, or agents which can modify, for example, the organoleptic properties.

4. Pharmaceutical composition according to one of Claims 1 to 3, **characterized in that** the aromatic group of the azetidine derivative of general formula (Ia) or (Ib) is chosen from a phenyl or naphthyl group.

5. Pharmaceutical composition according to one of Claims 1 to 4, **characterized in that** the aromatic group of the azetidine derivative of general formula (Ib) is a 3,5-difluorophenyl group.

6. Pharmaceutical composition according to one of Claims 1 to 3, **characterized in that** the heteroaromatic group is chosen from a pyridyl, furyl, thienyl, thiazolyl, imidazolyl or oxazolyl group.

7. Pharmaceutical composition according to one of Claims 1 to 6, **characterized in that** the nonionic surfactant with a hydrophilic character capable of solubilizing the azetidine derivative of general formula (Ia) or (Ib) and, where appropriate, the active ingredient potentiating the effects of the azetidine derivative, and capable of causing the formation of a colloidal system, is chosen from glycerides of polyethylene glycol and saturated fatty acids, whose HLB is between 10 and 20.

8. Pharmaceutical composition according to Claim 7, **characterized in that** the glycerides of polyethylene glycol and saturated fatty acids are glycerides of polyethylene glycol and saturated fatty acids containing from 6 to 18 carbon atoms.

9. Pharmaceutical composition according to Claim 7 or 8, **characterized in that** the glycerydes may be of natural or synthetic origin.

10. Pharmaceutical composition according to one of Claims 1 to 9, **characterized in that** the excipient with a lipophilic character, stabilizing the composition, is chosen from glycerides of polyethylene glycol and unsaturated fatty acids, from esters of polyethylene glycol and fatty acids or from esters of fatty acids and sorbitol, having an HLB of less than 10.

11. Pharmaceutical composition according to one of Claims 1 to 10, **characterized in that** the system comprising at most 2 excipients consists of Labrasol®, Gelucire® or the Labrafil®/Labrasol® pair.

12. Pharmaceutical composition according to one of Claims 1 to 11, **characterized in that** the active ingredient derived from azetidine, defined in Claim 1, is present in an amount of 0.01 to 70% by weight of the total composition.

13. Pharmaceutical composition according to one of Claims 1 to 12, **characterized in that** the nonionic surfactant with a hydrophilic character capable of solubilizing the azetidine derivative according to Claim 1, and capable of causing the formation of a colloidal system, is present in an amount of 20 to 100% relative to the total weight of the excipients in the composition.

14. Pharmaceutical composition according to one of Claims 1 to 13, **characterized in that,** where appropriate, agent with a lipophilic character, stabilizing the formulation, is present in an amount of 0.1 to 60% relative to the total weight of the excipients in the composition.

15. Pharmaceutical composition according to one of Claims 1 to 14, **characterized in that:**
• the active ingredient derived from azetidine is as defined in Claim 5,
• the nonionic surfactant with a hydrophilic character is Labrasol,
• the surfactant with a lipophilic character having an HLB of less than 10 is Labrafil M1944CS, and
• the Labrasol/Labrafil M1944CS mixture is present in a 60/40 (m/m) ratio relative to the total weight of the excipients present in the composition.

16. Pharmaceutical composition according to one of Claims 1 to 15, **characterized in that** it is provided in the form of a soft capsule.

17. Process for preparing a composition according to one of Claims 1 to 15, **characterized in that** there is prepared, where appropriate, the mixture of principal excipients, after heating, if necessary, in the case of the solid or semisolid excipients, and then, if necessary, the mixture with the additional additives, and then the azetidine derivative as defined in Claim 1 and, where appropriate, the active ingredient capable of potentiating the effects of the azetidine derivative of general formula (Ia) or (Ib), defined in Claim 1 are added and stirring is maintained in order to obtain a homogeneous mixture.

18. Presentation kit containing a composition according to Claim 3 and a composition comprising an active ingredient capable of potentiating the effects of the azetidine derivative defined in Claim 1.

19. Presentation kit according to Claim 18, containing a composition according to Claim 3 and a composition comprising sibutramine.

20. Presentation kit according to Claim 18, containing a composition according to Claim 3 and a composition comprising an agent which activates dopaminergic neurotransmission in the brain.

## Patentansprüche

1. Stabile pharmazeutische Zusammensetzung, umfassend mindestens ein Azetidinderivat der allgemeinen Formel: 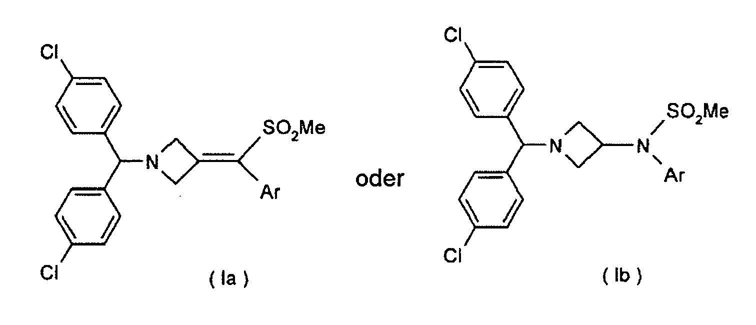 worin Ar für eine gegebenenfalls durch eine oder mehrere (C1-C4)-Alkyl-, Halogen-, NO₂-, CN-, (C1-C4)-Alkoxy- oder OH-Gruppen substituierte aromatische oder heteroaromatische Gruppe steht, gegebenenfalls in Kombination mit einem anderen Wirkstoff, der zur Potenzierung der Wirkungen des Azetidinderivats der allgemeinen Formel (Ia) oder (Ib) befähigt ist, in einem System, das höchstens 2 Haupthilfsstoffe umfaßt, die unter einem nichtionischen Tensid mit hydrophilem Charakter, das zur Solubilisierung des Azetidinderivats der allgemeinen Formel (Ia) oder (Ib) und gegebenenfalls des die Wirkungen des Azetidinderivats potenzierenden Wirkstoffs befähigt ist und zur Bewirkung der Bildung eines kolloidalen Systems befähigt ist, und gegebenenfalls einem die Formulierung stabilisierenden zweiten Hilfsstoff lipophiler Natur ausgewählt sind.

2. Pharmazeutische Zusammensetzung nach Anspruch 1, **dadurch gekennzeichnet, daß** sie:
■ mindestens ein Azetidinderivat der allgemeinen Formel (Ia) oder (Ib) gemäß Anspruch 1,
■ gegebenenfalls einen anderen Wirkstoff, der zur Potenzierung der Wirkungen des Azetidinderivats der allgemeinen Formel (Ia) oder (Ib) befähigt ist,
■ ein nichtionisches Tensid mit hydrophilem Charakter, das zur Solubilisierung des Azetidinderivats der allgemeinen Formel (Ia) oder (Ib) und gegebenenfalls des die Wirkungen des Azetidinderivats potenzierenden Wirkstoffs befähigt ist und zur Bewirkung der Bildung eines kolloidalen Systems befähigt ist,
■ gegebenenfalls ein Tensid mit lipophilem Charakter, das einen HLB-Wert von weniger als 10 aufweist und die Zusammensetzung stabilisiert,
■ gegebenenfalls zusätzliche Additive, ausgewählt unter Stabilisierungsmitteln, Konservierungsmitteln, Mitteln, die die Einstellung der Viskosität ermöglichen, oder Mitteln, die beispielsweise die organoleptischen Eigenschaften modifizieren können,
umfaßt.

3. Pharmazeutische Zusammensetzung nach Anspruch 1 oder 2, **dadurch gekennzeichnet, daß** sie:
■ mindestens ein Azetidinderivat der allgemeinen Formel (Ia) oder (Ib) gemäß Anspruch 1,
■ ein nichtionisches Tensid mit hydrophilem Charakter, das zur Solubilisierung des Azetidinderivats gemäß Anspruch 1 befähigt ist und zur Bewirkung der Bildung eines kolloidalen Systems befähigt ist,
■ gegebenenfalls ein Tensid mit lipophilem Charakter, das einen HLB-Wert von weniger als 10 aufweist und die Zusammensetzung stabilisiert,
■ gegebenenfalls zusätzliche Additive, ausgewählt unter Stabilisierungsmitteln, Konservierungsmitteln, Mitteln, die die Einstellung der Viskosität ermöglichen, oder Mitteln, die beispielsweise die organoleptischen Eigen■ schaften modifizieren können,
umfaßt.

4. Pharmazeutische Zusammensetzung nach einem der Ansprüche 1 bis 3, **dadurch gekennzeichnet, daß** die aromatische Gruppe des Azetidinderivats der allgemeinen Formel (Ia) oder (Ib) unter einer Phenyl- oder Naphthylgruppe ausgewählt ist.

5. Pharmazeutische Zusammensetzung nach einem der Ansprüche 1 bis 4, **dadurch gekennzeichnet, daß** es sich bei der aromatischen Gruppe des Azetidinderivats der allgemeinen Formel (Ib) um eine 3,5-Difluorphenylgruppe handelt.

6. Pharmazeutische Zusammensetzung nach einem der Ansprüche 1 bis 3, **dadurch gekennzeichnet, daß** die heteroaromatische Gruppe unter einer Pyridyl-, Furyl-, Thienyl-, Thiazolyl-, Imidazolyl- oder Oxazolylgruppe ausgewählt ist.

7. Pharmazeutische Zusammensetzung nach einem der Ansprüche 1 bis 6, **dadurch gekennzeichnet, daß** das nichtionische Tensid mit hydrophilem Charakter, das zur Solubilisierung des Azetidinderivats der allgemeinen Formel (Ia) oder (Ib) und gegebenenfalls des die Wirkungen des Azetidinderivats potenzierenden Wirkstoffs befähigt ist und zur Bewirkung der Bildung eines kolloidalen Systems befähigt ist, unter Glyceriden von Polyethylenglykol und gesättigten Fettsäuren mit einem HLB-Wert zwischen 10 und 20 ausgewählt ist.

8. Pharmazeutische Zusammensetzung nach Anspruch 7, **dadurch gekennzeichnet, daß** es sich bei den Glyceriden von Polyethylenglykol und gesättigten Fettsäuren um Glyceride von Polyethylenglykol und gesättigten Fettsäuren mit 6 bis 18 Kohlenstoffatomen handelt.

9. Pharmazeutische Zusammensetzung nach Anspruch 7 oder 8, **dadurch gekennzeichnet, daß** die Glyceride natürlicher oder synthetischer Herkunft sein können.

10. Pharmazeutische Zusammensetzung nach einem der Ansprüche 1 bis 9, **dadurch gekennzeichnet, daß** der die Formulierung stabilisierende Hilfsstoff mit lipophilem Charakter unter Glyceriden von Polyethylenglykol und ungesättigten Fettsäuren, Estern von Polyethylenglykol und Fettsäuren oder Estern von Fettsäuren und Sorbitol mit einem HLB-Wert von weniger als 10 ausgewählt ist.

11. Pharmazeutische Zusammensetzung nach einem der Ansprüche 1 bis 10, **dadurch gekennzeichnet, daß** das höchstens 2 Hilfsstoffe umfassende System aus Labrasol,®, Gelucire® oder dem Paar Labrafil®/Labrasol® besteht.

12. Pharmazeutische Zusammensetzung nach einem der Ansprüche 1 bis 11, **dadurch gekennzeichnet, daß** der von Azetidin abgeleitete Wirkstoff gemäß Anspruch 1 in einer Menge von 0,01 bis 70 Gew.-%, bezogen auf die gesamte Zusammensetzung, vorliegt.

13. Pharmazeutische Zusammensetzung nach einem der Ansprüche 1 bis 12, **dadurch gekennzeichnet, daß** das nichtionische Tensid mit hydrophilem Charakter, das zur Solubilisierung des Azetidinderivats gemäß Anspruch 1 befähigt ist und zur Bewirkung der Bildung eines kolloidalen Systems befähigt ist, in einer Menge von 20 bis 100 Gew.-%, bezogen auf das Gesamtgewicht der Hilfsstoffe in der Zusammensetzung, vorliegt.

14. Pharmazeutische Zusammensetzung nach einem der Ansprüche 1 bis 13, **dadurch gekennzeichnet, daß** gegebenenfalls Mittel mit lipophilem Charakter, das die Formulierung stabilisiert, in einer Menge von 0,1 bis 60 Gew.-%, bezogen auf das Gesamtgewicht der Hilfsstoffe in der Zusammensetzung, vorliegt.

15. Pharmazeutische Zusammensetzung nach einem der Ansprüche 1 bis 14, **dadurch gekennzeichnet, daß:**
■ der von Azetidin abgeleitete Wirkstoff wie in Anspruch 5 definiert ist,
■ es sich bei dem nichtionischen Tensid mit hydrophilem Charakter um Labrasol handelt,
■ es sich bei dem Tensid mit lipophilem Charakter und einem HLB-Wert von weniger als 10 um Labrafil M1944CS handelt und
■ die Mischung aus Labrasol und Labrafil M1944CS in einem Verhältnis von 60/40 (m/m), bezogen auf das Gesamtgewicht der Hilfsstoffe in der Zusammensetzung, vorliegt.

16. Pharmazeutische Zusammensetzung nach einem der Ansprüche 1 bis 15, **dadurch gekennzeichnet, daß** sie in Form einer Weichkapsel vorliegt.

17. Verfahren zur Herstellung einer Zusammensetzung gemäß einem der Ansprüche 1 bis 15, **dadurch gekennzeichnet, daß** man gegebenenfalls die Mischung von Haupthilfsstoffen, im Fall von festen oder halbfesten Hilfsstoffen gegebenenfalls nach Erhitzen, und dann gegebenenfalls die Mischung mit den zusätzlichen Additiven herstellt, dann das Azetidinderivat gemäß Anspruch 1 und gegebenenfalls den Wirkstoff, der zur Potenzierung der Wirkungen des Azetidinderivats der allgemeinen Formel (Ia) oder (Ib) gemäß Anspruch 1 befähigt ist, zugibt und dann unter Rühren hält, um eine homogene Mischung zu erhalten.

18. Darreichungskit, enthaltend eine Zusammensetzung gemäß Anspruch 3 und eine Zusammensetzung, die einen Wirkstoff, der zur Potenzierung der Wirkungen des Azetidinderivats gemäß Anspruch 1 befähigt ist, umfaßt.

19. Darreichungskit nach Anspruch 18, enthaltend eine Zusammensetzung gemäß Anspruch 3 und eine Zusammensetzung, die Sibutramin umfaßt.

20. Darreichungskit nach Anspruch 18, enthaltend eine Zusammensetzung gemäß Anspruch 3 und eine Zusammensetzung, die ein Mittel, das die dopaminerge Neurotransmission im Gehirn aktiviert, umfaßt.
